# EUROPEAN PATENT SPECIFICATION

(11) **EP 3 661 448 B1**
(45) Date of publication and mention of the grant of the patent: **17.02.2021**
(21) Application number: 18755379.7
(22) Date of filing: 31.07.2018
(51) Int. Cl.: A61L 15/58, A61B 50/30, A61F 13/36

(54) **SYSTEMS FOR WOUND DEBRIDEMENT**
SYSTEME FÜR WUNDDEBRIDEMENT
SYSTÈMES DE DÉBRIDEMENT DE PLAIE

(30) Priority: 02.08.2017 US 201762540423 P
(43) Date of publication of application: 10.06.2020
(73) Proprietor: 3M Innovative Properties Company, St. Paul, MN 55133-3427 (US)
(72) Inventor: ROBINSON, Timothy, Mark, Shillingstone DT11 0TG (GB); LOCKE, Christopher, Brian, Bournemouth BH9 3SD (GB)
(74) Representative: Simmons & Simmons
(86) International application number: PCT/US2018/044661
(87) International publication number: WO 2019/028057

(56) References cited:
- WO-A1-95/04510
- WO-A1-2013/180832
- US-A- 5 203 764
- US-A- 5 843 060
- US-A1- 2010 030 171
- US-A1- 2016 331 860

## Description

### Technical Field

This disclosure relates generally to treatment of wounds and, in particular, to materials for debriding a wound.

### Background

Debridement is a well-known and important procedure of removing undesired (e.g., dead, damaged, infected, or the like) tissue from a wound to promote the healing of healthy tissue in and around the wound. While surgical (e.g., scalpel, laser, etc.) and mechanical (e.g., irrigation, suction, hand tools, etc.) debridement are most commonly employed, it is not uncommon for fabric-based wound cleaning materials (e.g., pads, cloths, etc.) to be used for manual cleaning and debridement of a wound when surgical or mechanical debridement is not practical.

Fabric-based wound cleaning materials are advantageous in that they are low cost and reasonably effective to manually debride a wound. A major challenge presented by such fabric-based wound debriding materials is that they are prone to either partially disintegrating or otherwise leaving some of its material (e.g., fibers) in the wound during debridement, especially when they are used to debride tough (e.g., necrotic) tissues. This requires the caregiver (e.g., a nurse, a doctor, or the like) to incorporate an additional step of flushing the wound (e.g., with water, saline, or the like) with the hope that the flushing would wash away any fibers or polymeric material left in the wound. However, flushing the wound is not always effective to wash away all of the small fabric and/or polymer materials from the surface of the wound and/or around the wound. As a result, some fibers of the debriding material may be undesirably left in the wound, potentially impeding the healing of the wound and/or leading to an infection. US5,203,764 discloses an open-cell foam pad impregnated with water curable, isocyanate functional, polyurethane prepolymer resin.
US 5,843,060 discloses nasal, sinus, and otic packings having a less adherent surface to be less traumatic on removal.
US2010/0030171 discloses a composite article having a fluid transport layer, a fluid retentive layer, and a moisture vapor permeable film for use as a wound dressing. US2016/0331860 discloses a wet wound dressing formed from hydrophilic polyurethane foam. WO95/04510 discloses a device for wound treatment of stage II - IV wounds comprising a surgical dressing of a polyvinyl acetal having a pore diameter ranging from about 0.3 mm to about 1.0 mm.
WO2013/180832 discloses an absorbent article having a polymeric foam with an average cell size of at least 100 microns, and an indentation force of less than 75 N at 50%.

### Brief Description of the Drawings

Disclosed herein are embodiments of systems, apparatuses, methods, and systems pertaining to wound debridement using wound debridement materials. This description includes drawings, wherein:
FIG. 1 is a perspective view of a wound debriding material in accordance with some embodiments;
FIG. 2 illustrates a step of an exemplary embodiment of a process of debriding a wound surface of a patient, depicting the debriding material of FIG. 1 being grasped by a user and brought by the user in a direction toward the wound surface to be debrided;
FIG. 3 illustrates a step of an exemplary embodiment of a process of debriding a wound surface of a patient, depicting the user holding the debriding material of FIG. 1 and applying the debriding material across at least a portion of the wound surface;
FIG. 4 illustrates the wound surface of the patient and the debriding material of FIG. 1 after the wound surface has been debrided as shown in FIG. 4, depicting the debriding material being stained by tissue picked up from the wound surface during the debriding and the wound surface after the debridement and being free of debris from the debriding material; and
FIG. 5 illustrates a perspective view of a package containing the wound debriding material of FIG. 1 in accordance with some embodiments; and
FIG. 6 illustrates a flow diagram of a method of facilitating debridement of a wound of a patient in accordance with some embodiments.

Elements in the figures are illustrated for simplicity and clarity and have not been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments of the present invention. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein.

### Detailed Description

The following description is not to be taken in a limiting sense, but is made merely for the purpose of describing the general principles of exemplary embodiments. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

Generally, pursuant to various embodiments, materials are provided for wound debridement. In some embodiments not according to the invention, a method of debriding a wound of a patient includes applying a wound debridement material in frictional contact across at least a portion of a wound surface of the patient. The wound surface includes tissue to be removed and the wound debridement material includes a solid porous foam soaked by a fluid. The method further includes removing, at least a portion of the tissue from the wound surface via adherence of the at least the portion of the tissue to the wound debridement material without leaving solid debris of the foam on the wound surface after the applying step. Without wishing to be limited by theory, the tissue is removed from the surface of the wound as a result of friction between the surface of the wound debridement material and the surface of the wound that contains tissue to be removed and/or due to the wetness of the wound debridement material that facilitates the adherence of at least some of the tissue to the wound debridement material and/or via absorption of at least some of the tissue, exudate, or the like from the wound surface into the surface of the wound debridement material.

In some embodiments not according to the invention, a method of facilitating debridement of a wound of a patient includes: providing a package including a wound debridement material comprising a solid porous foam soaked by a fluid such that the moisture content of the foam is between about 20 wt. % and about 60 wt. %; and providing instructions, on the package, instructing a user to apply the wound debridement material across at least a portion of a wound surface of the patient in order to remove tissue from the wound surface via adherence of the tissue to the wound debridement material.

In some embodiments not according to the invention, a method of making a packaged wound debridement material for debriding a wound of a patient includes: providing a wound debridement material comprising a solid porous foam; soaking the foam in a fluid such that the moisture content of the foam is between about 20 wt. % and about 60 wt. %; inserting the soaked foam into a package; and sealing the package. The present invention concerns a wound debridement material as claimed in claim 1. Preferred embodiments are claimed in dependent claims 2-5.

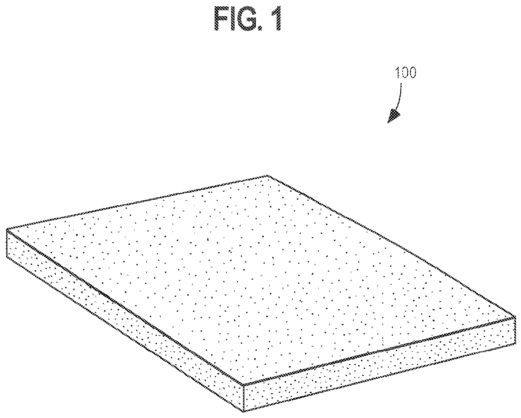

FIG. 1 illustrates an exemplary wound debridement material 100 that may be used to debride a wound of a patient and remove tissue from the surface and around the wound of the patient via adherence of the tissue to the wound debridement material 100 and without leaving solid debris of the wound debridement material 100 on or around the wound surface. As used herein, the terms "debride," "debriding," and "debridement," refer to removal of undesirable (e.g., eschar, necrotic, damaged, infected, or the like) tissue or a foreign material from a wound of a patient. The term patient will be understood to include both a human patient and an animal patient.

The wound debridement material 100 illustrated in FIG. 1 is a solid porous foam. In some aspects, the wound debridement material 100 is an open-celled foam, for example a reticulated open-celled foam or a non-reticulated open-celled foam. Generally, the term "cells" refer to interconnecting compartments within a foam to form a structural network and the term "open-celled foam" refers to a foam having interconnected cells or a net-like microstructure with few, if any, closed cells such that the cells are substantially continuous between the opposing external surfaces of the foam and have openings at the external surfaces. Conversely, the term "closed-celled foam" generally refers to foams that do not have interconnected cells and that do not have openings at the external surfaces of the foam.

In some embodiments, the wound debridement material 100 is WhiteFoam® (available from Kinetic Concepts, Inc. of San Antonio, Texas), which is a white, open-celled, non-reticulated polyvinyl alcohol-based solid foam having a hydrophilic (i.e., moisture-retaining) structure and pores having a diameter from about 200 to about 1000 microns. In certain embodiments, the wound debridement material 100 is formed of a material including but not limited to: polyvinyl alcohol (PVA); polyurethanes (e.g., polyurethane-polyester, polyurethane-polyether, or the like); polyolefins (e.g., polypropylenes (PP), polyethylenes (PE), or the like), silicone elastomers and copolymers; polyvinylchloride (PVC); polyamides; polyesters; acrylics; thermoplastic elastomers (e.g., styrene-butene-styrene (SBS), styrene-ethylene-butene-styrene (SEBS), or the like); polyether-amide block copolymers (PEBAX); elastomers (e.g., styrene butadiene rubber (SBR); ethylene propylene rubber (EPR); ethylene propylene diene modified rubber (EPDM); natural rubber (NR); vinyl acetate, ethylene vinyl acetate (EVA); polyvinyl acetal; polyvinyl butyral (PVB), or the like).

In some aspects, the wound debridement material 100 of FIG. 1 is dimensioned such that it has a length of about 10 centimeters, a width of about 7.5 centimeters, and a height or thickness of about 1 centimeter. It will be appreciated that these dimensions are provided by way of example only, and that the wound debridement material 100 of FIG. 1 may have various dimensions depending on the size of the wound being debrided. For example, in some aspects, the wound debridement material may have a length from about 5 to about 20 centimeters, a width from about 3 to about 15 centimeters, and a thickness of about 0.5 to about 2 centimeters.

In certain aspects, the porosity of the solid foam of FIG. 1 can range from about 95% to about 98%, but it will be appreciated that less porous (e.g., from about 80% to about 95%) or more porous (e.g., from about 98% to about 100%) solid foams may be used. In some embodiments, the wound debridement material 100 is formed from a plurality of fibers each having a diameter from about 1 micron to about 100 microns, and in some aspects, from about 5 microns to about 50 microns.

The wound debridement material 100 has a density from 145 kg/m³ to 270 kg/m³. The wound debridement material 100 has a stiffness of 30 Shore A durometer to 70 Shore A durometer. In some aspects, the wound debridement material 100 is mechanically compressed to increase its density at ambient pressure. The compression stiffness of the would debridement material 100 is from 4.0 kPa at 25% compression to 9.0 kPa at 50% compression.

Generally, a compressed foam may be characterized by a firmness factor (FF) that is defined as a ratio of the density of a foam in a compressed state to the density of the foam in an uncompressed state. For example, a firmness factor (FF) of 5 may refer to a compressed foam having a density that is five times greater than a density of the same foam in an uncompressed state. Mechanically or chemically compressing a foam may reduce a thickness of the foam at ambient pressure when compared to the same foam that has not been compressed. Reducing a thickness of a foam by mechanical or chemical compression may increase a density of the foam, which may increase the firmness factor (FF) of the foam. In some embodiments, the wound debridement material 100 is configured to absorb moisture (e.g., exudate, pus, blood, etc.) and/or remove up dry tissue from a wound of a patient by drawing or wicking such moisture and/or lifting (e.g., by adherence to the moist surface(s) of the wound debridement material 100) the dry tissue up from the surface of the wound and/or around the wound.

The wound debridement material 100 is a foam having a moisture content from 20 wt. % to 60 wt. %. Without wishing to be limited by theory, the moisture content of the foam in such embodiments provides the foam with a texture that permits the wound debridement material, applied in frictional contact (e.g., rubbing) across at least a portion of surface of a wound of a patient, to remove tissue from the surface of the wound and/or around the wound via friction between the surface of the wound debridement material 100 and the surface of the wound and/or due to the wetness of the wound debridement material 100 that facilitates the adherence of at least some of the dry and/or wet tissue to the wound debridement material 100 and/or via absorption of at least some of the tissue from the wound surface into the surface of the wound debridement material 100.

In some aspects, the fluid that provides the wound debridement material 100 with its moisture content is a liquid that includes but is not limited to: water, saline, polyhydric alcohol, mixtures thereof, or the like. Given that the wound debridement material 100 is configured for application to a wound of a patient in a medical setting, the liquid is sterile.

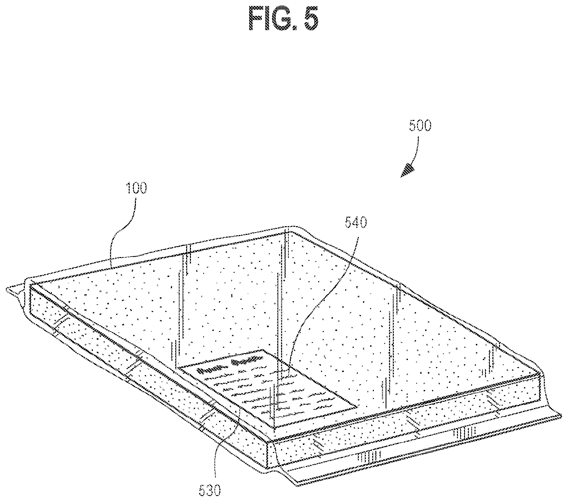

In some configurations, the wound debridement material 100 may be pre-moistened and retained in sealed packaging (e.g., package 500 of FIG. 5, which will be described in more detail below), such that a user would only be required to open the packaging before applying (e.g., by rubbing) the wound debridement material 100 across a portion of a wound (as will be discussed in more detail below). In certain aspects, the moisture content of the wound debridement material 100 may be adjusted depending on the nature of the wound to be debrided.

For example, a wound debridement material 100 selected to debride a wound having a greater amount of harder tissue (e.g., eschar) to be removed would be adjusted to have a lower moisture content (e.g., from about 20 wt. % to about 40 wt. %).Conversely, a wound debridement material 100 selected to debride a wound having a comparatively lesser amount of harder tissue to be removed would be adjusted to have a higher moisture content (e.g., from about 40 wt. % to about 60 wt. %). In other words, a wound debridement material 100 having a lower moisture percentage is likely to be more effective in debriding a wound having more dry, harder tissue than a wound debridement material 100 having a comparatively higher moisture percentage.

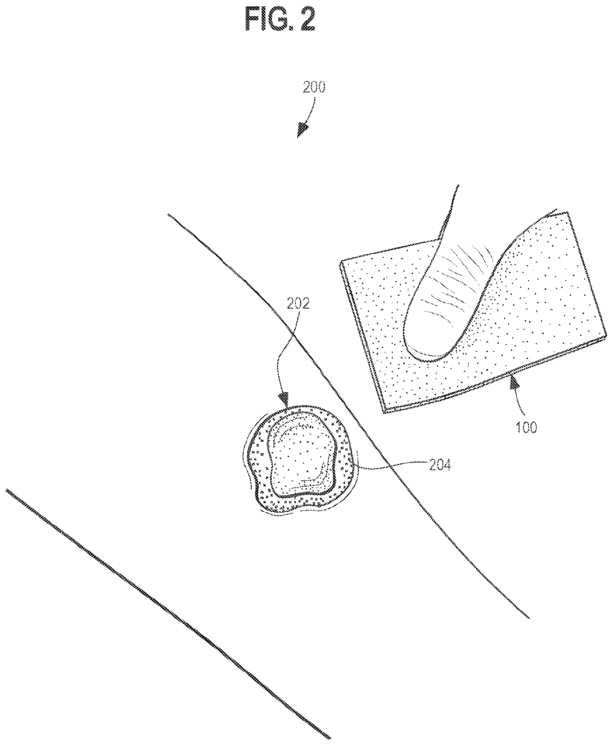

With reference to FIGS. 2-4, an exemplary method 200 of debriding a wound 202 of a patient is illustrated. As can be seen in FIG. 2, the surface of the wound 202 includes tissue 204 to be removed. It will be appreciated that at least some of the tissue 204 to be removed via the method 200 is located around the surface of the wound 202. As described above, the wound debridement material 100 comprises a solid porous foam (e.g., a polyvinyl alcohol foam) soaked by a fluid. As discussed above, in some embodiments, the fluid provides the wound debridement material 100 with a moisture content of about 20 wt. % to about 60 wt. %.

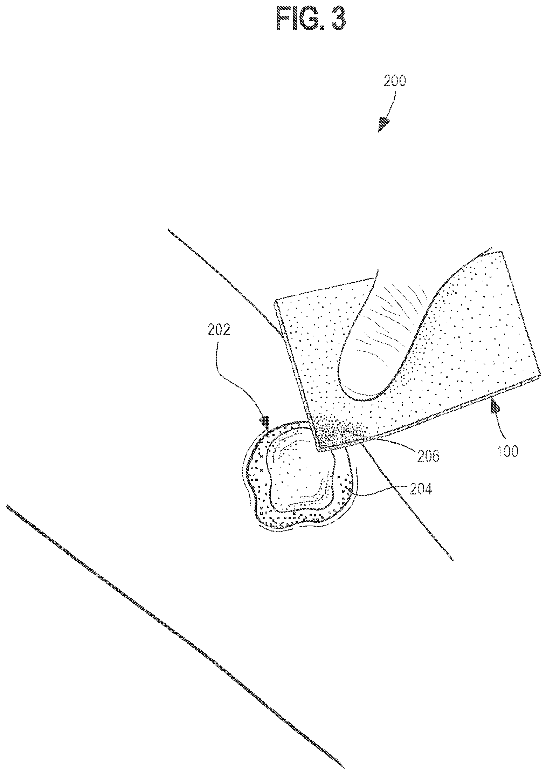

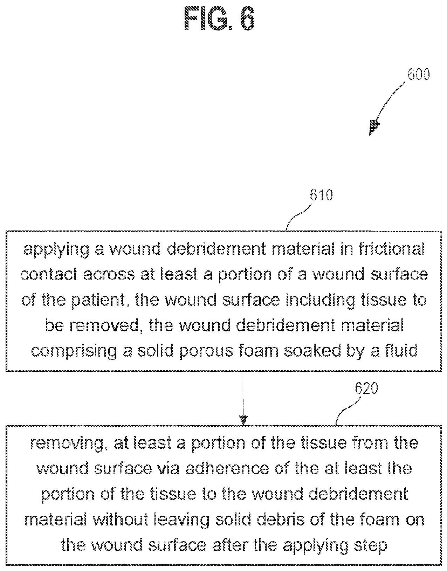

As can be seen in FIG. 2, one step of the method 200 includes a user (e.g., a nurse, doctor, or the like) grasping the wound debridement material 100 and bringing the wound debridement material 100 in a direction toward the wound 202 of the patient. With reference to FIG. 3, one step of the method 200 includes applying (e.g., rubbing) the wound debridement material 100 in frictional contact across at least a portion of the surface of the wound 202 of the patient. As can be seen in FIG. 6, which shows a diagrammatic representation of the method illustrated in FIGS. 2-4, the method 600 includes a step 610 of applying a wound debridement material 100 in frictional contact across at least a portion of a surface of a wound 202 of the patient, the wound surface including tissue 204 to be removed, the wound debridement material 100 comprising a solid porous foam soaked by a fluid.

FIG. 3 illustrates another step of the method 200 and, more specifically, the removing of at least a portion of the tissue 204 from the surface of the wound 202 of the patient via the wound debridement material 100. As can be seen in FIG. 6, which shows a diagrammatic representation of the method shown in FIGS. 2-4, the method 600 includes a step 620 of removing, at least a portion of the tissue 204 from the surface of the wound 202 via adherence of the at least the portion of the tissue 204 to the wound debridement material 100 without leaving solid debris of the foam on the surface of the wound 204 after the applying step.

As mentioned above, the wound debridement material 100 is configured (e.g., by having a moisture level within a predetermined range and by having a suitable texture as a result having such a moisture level) to remove tissue from the surface of the wound 202 and/or around the wound 2020 via frictional contact (e.g., achieved by a user rubbing the wound debridement material 100 across the surface of the wound 202) between the surface of the wound debridement material 100 and the surface of the wound and/or due to the wetness of the wound debridement material 100 that facilitates the adherence of at least some of the dry and/or wet tissue to the wound debridement material 100 and/or via absorption of at least some of the tissue from the wound surface into the surface of the wound debridement material 100.

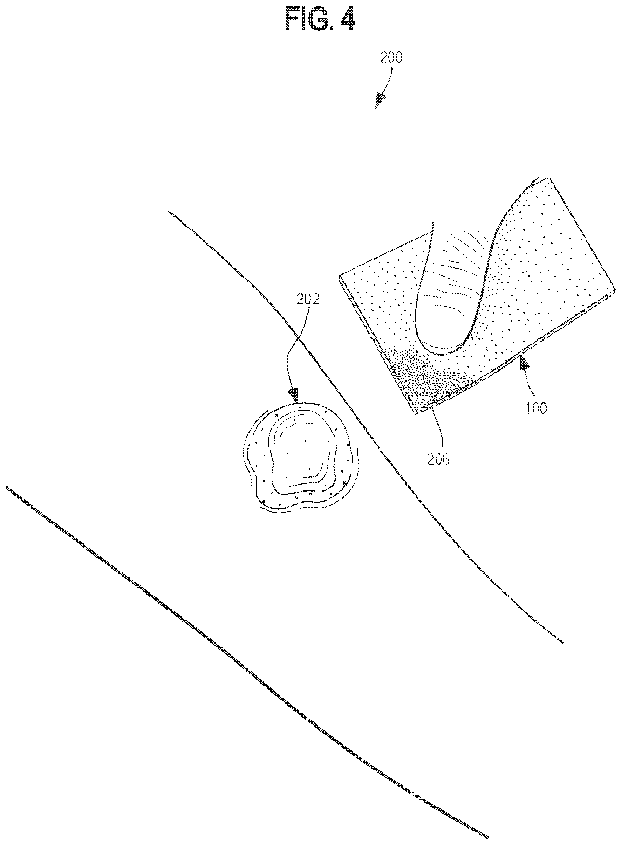

In the exemplary embodiment illustrated in FIGS. 3-4, the tissue 204 (e.g., dry eschar, exudate, etc.) is removed from the surface of the wound 202 as a result of friction between the surface of the wound debridement material 100 and the surface of the wound 202, as well as via adherence (e.g., by sticking and/or absorption) of at least some of the tissue 204 to the wound debridement material 100. For example, FIGS. 3 and 4 show the staining 206 (i.e., depicted by darker regions) of the wound debridement material 100 caused by adherence of the tissue 204 to the wound debridement material 100 and/or absorption of at least a portion of the tissue 204 into the surface of the wound debridement material 100.

FIG. 4 also illustrates that as a result of the wound debridement material 100 being applied to (e.g., rubbed on) the surface of the wound 202 to debride the wound 202 as described above, no solid debris of the wound debridement material 100 are left on or around the surface of the wound 202. In other words, the wound debridement material 100 advantageously has a construction that does not fall apart and does not have pieces (e.g., fibers) of it break off as a result of frictional contact and movement of the wound debridement material 100 relative to the (often dry, hard) tissue 204 that is debrided on and around a wound 202.

With reference to FIG. 5, in certain embodiments, a method of facilitating debridement of a wound of a patient includes providing a package 500 including wound debridement material 100 comprising a solid porous foam soaked by a fluid such that the moisture content of the foam is between about 20 wt. % and about 60 wt. %. In some aspects, the method includes providing wound debridement instructions to a user on the package. In the embodiment of FIG. 5, the debridement instructions are included on the package 500 in the form of a label 530 affixed to the package 500 and including written instructions 540 to a user. It will be appreciated that the use of the label 530 is shown in FIG. 5 by way of example only, and that the user instructions may be printed directly on the material (e.g., polymer film) that forms the package 530. Similarly, the location of the label and instructions are shown by way of example only in FIG. 5, and may be provided in a different location or on an opposite face of the package 500.

In one approach, the method includes instructing a user to apply (e.g., rub) the wound debridement material 100 in frictional contact across at least a portion of a surface of a wound 202 of the patient in order to remove tissue 204 from the surface of the wound 202 via adherence of the tissue 204 to the wound debridement material 100. In some aspects, the package 500 includes a notification that the application of the wound debridement material 100 to at least a portion of the surface of the wound 202 of the patient to remove at least a portion of the tissue 204 from the surface of the wound 202 via adherence of the tissue 204 to the wound debridement material 100 will not leave solid debris of the wound debridement material 100 on or around the surface of the wound 202.

In some embodiments, the package 500 containing the wound debridement material 100 is hermetically sealed such that the wound debridement material 100 is sealed and soaked by a fluid as described above and retains its initial moisture content due to being sealed and not exposed to ambient air while in the package 500. In such embodiments, prior to the application of the wound debridement material 100 across at least a portion of the surface of the wound 202, the method of debriding the wound 202 of the patient further includes removing the wound debridement material 100 from a sealed package 500 containing the wound debridement material 100 soaked by the fluid.

In some embodiments, the wound debridement material 100 is soaked with the fluid in the sealed package 500 such that the wound debridement material 100 has a moisture content of about 20 % to about 60 % by total weight of the wound debridement material 100. In such embodiments, after the opening of the package 500 (e.g., by tearing away a portion of the package 500) and the removal of the wound debridement material 100 from the package 500, the next step of the method of debriding the wound 202 would include applying (e.g., by rubbing) the wound debridement material 100 removed from the package 500 in frictional contact across at least a portion of the surface of the wound 202 of the patient, as described above, to remove at least a portion of the tissue 204 from the surface of the wound 202 via adherence of the tissue 204 to the wound debridement material 100 and without leaving solid debris of the wound debridement material 100 on or around the surface of the wound 202.

In some embodiments, the wound debridement material 100 is soaked with the fluid in the package 500 such that the wound debridement material 100 has a moisture content of above 60 wt. % (e.g., from about 60 wt. % to about 100 wt. %). Since the wound debridement material 100, when soaked with a fluid such that it has a moisture above 60 wt. % does not possess optimal surface friction coefficient for debriding the tissue 204 from the surface of the wound 202, in such embodiments, after the removal of the wound debridement material 100 from the package 500, the next step of the method of debriding the wound 202 would include removing at least a portion of the fluid absorbed in the wound debridement material 100 until the wound debridement material 100 has a moisture content between about 20 wt. % and about 60 wt. %. In some aspects, the fluid may be removed from the wound debridement material 100 simply by squeezing (or otherwise applying pressure to) the wound debridement material 100 such that the fluid is ejected therefrom.

In some embodiments, after fluid is removed from the wound debridement material 100 to bring the moisture level of the wound debridement material to a moisture percentage with the range of about 20 wt. % to about 60 wt. %, the next step of the method of debriding the wound 202 would include applying (e.g., rubbing) the wound debridement material 100 removed from the package 500 (and having its moisture content decreased to a moisture content between about 20 wt. % and about 60 wt. %) in frictional contact across at least a portion of the surface of the wound 202 of the patient, as described above, thereby removing at least a portion of the tissue 204 from the surface of the wound via adherence of the tissue 204 to the wound debridement material 100 and without leaving solid debris of the wound debridement material 100 on or around the surface of the wound 202.

In some embodiments, the wound debridement material 100 is provided in the package 500 such that the wound debridement material 100 has a moisture content of 0, i.e., the wound debridement material 100 is completely dry. Given that the wound debridement material 100, when completely dry, has a hard texture and thus a friction coefficient that may lead to irritation of the surface of the wound 202 of the patient, in such embodiments, after the removal of the wound debridement material 100 from the package 500, the next step of the method of debriding the wound 202 would include applying a fluid (e.g., water, saline, polyhydric alcohol, or the like) to at least a portion of the wound debridement material 100 until the wound debridement material 100 has a moisture content between about 20 % and about 60 % based on total weight of the wound debridement material 100. In some aspects, the fluid is applied onto the wound debridement material 100 by pouring the fluid from a suitable container (e.g., bottle) or device. In other aspects, the fluid is applied onto the wound debridement material 100 by soaking the wound debridement material 100 in the fluid contained in a suitable receptacle (e.g., tub).

The next step of such a method would be applying (e.g., rubbing) the wound debridement material 100 removed from the package 500 (and having its moisture content increased to a moisture content between about 20 wt. % and about 60 wt. %) in frictional contact across at least a portion of the surface of the wound 202 of the patient, as described above, to remove at least a portion of the tissue 204 from the surface of the wound via adherence of the tissue 204 to the wound debridement material 100 and without leaving solid debris of the wound debridement material 100 on surface of the wound 202.

In some embodiments, the wound debridement material 100 is applied (e.g., placed into direct contact and rubbed) across at least a portion of the wound surface of the patient at a pressure of about 100 kPa to about 150 kPa. Generally, the pressure at which the wound debridement material 100 is applied to the surface of the wound 202 can be adjusted based on the moisture content of the wound debridement material 100. In other words, in some aspects, a wound debridement material 100 having a higher moisture content (e.g., from about 40 wt. % to about 60 wt. %) would be applied to the surface of the wound 202 at a higher pressure (e.g., from about 125 kPa to about 150 kPa). Conversely, a wound debridement material 100 having a moisture content that is lower (e.g., from about 20 wt. % to about 40 wt. %) would be applied to the surface of the wound 202 at a lower pressure (e.g., from about 100 kPa to about 125 kPa).

In certain embodiments, a method of making a packaged wound debridement material 100 for debriding a wound of a patient is also provided. The exemplary method includes providing a wound debridement material 100 comprising a solid porous foam. As discussed above, the wound debridement material 100 may be a solid polyvinyl alcohol open-celled foam. The next step of such an exemplary method includes soaking the solid foam in a fluid such that the foam absorbs the fluid and achieves a moisture content of between about 20 % and about 60 % by total weight of the solid foam. The method then includes inserting the soaked solid foam into a package 500 and sealing the package 500. In some aspects, the soaked solid foam is hermetically sealed in the package 500.

In some aspects and with reference to FIG. 5, after the soaked wound debridement material 100 is sealed in a package 500, the method of making a packaged wound debridement material 100 for debriding a wound of a patient further includes providing instructions 530, on the package 500, in order to instruct a user to apply (e.g., rub) the wound debridement material 100 in frictional contact across at least a portion of and/or around a surface of a wound 202 of the patient in order to remove tissue 204 from the surface of the wound 202 via adherence of the tissue 204 to the wound debridement material 100. As described above and shown in FIG. 5, in some aspects, the package 500 includes a notification (e.g., label) 530 that the application of the wound debridement material 100 to at least a portion of the surface of the wound 202 of the patient to remove at least a portion of the tissue 204 from the surface of the wound 202 via adherence of the tissue 204 to the wound debridement material 100 will not leave solid debris of the wound debridement material 100 on or around the surface of the wound 202.

The methods and materials described herein facilitate debridement of wounds of patients without leaving solid pieces of the wound debridement materials in or on the wound. This advantageously reduces infections following surgical procedures and obviates the need for medical personnel to take extra steps in washing a wound surface following a surgical procedure.

## Claims

1. A wound debridement material comprising:
a solid open-cell hydrophilic porous foam (100) soaked by a liquid such that the foam has a moisture content from 20 wt. % to 60 wt. %;
wherein the solid porous foam (100) has a texture that permits the wound debridement material, when applied in frictional contact across at least a portion of a wound surface of a patient including tissue to be removed, to remove the tissue from the wound surface via adherence of the tissue to the wound debridement material without leaving solid debris of the foam on the wound surface; **characterised in that**
the foam has a stiffness of 30 Shore A durometer to 70 Shore A durometer;
a compression stiffness of the foam is from 4.0 kPa at 25% compression to 9.0 kPa at 50% compression; and
a density from 145kg/m3 to 270 kg/m3.

2. The wound debridement material of claim 1, wherein the foam is a polymer selected from the group consisting of polyvinyl alcohol, polyurethane, polyolefin, vinyl acetate, silicone elastomers, and their copolymers.

3. The wound debridement material of claim 1, wherein the foam comprises a plurality of fibers each having a diameter from 5 microns to 50 microns.

4. The wound debridement material of claim 1, wherein the liquid is selected from the group consisting of water, saline, and a polyhydric alcohol, and mixtures thereof.

5. The wound debridement material of claim 1, wherein the liquid is sterile.

## Patentansprüche

1. Wundreinigungsmaterial, umfassend:
einen festen, offenzelligen, hydrophilen, porösen Schaumstoff (100), der mit einer Flüssigkeit getränkt ist, sodass der Schaumstoff einen Feuchtigkeitsgehalt von 20 Gew.-% bis 60 Gew.-% aufweist;
wobei der feste poröse Schaumstoff (100) eine Textur aufweist, die dem Wundreinigungsmaterial gestattet, wenn es über mindestens einem Abschnitt einer Wundoberfläche eines Patienten, der zu entfernendes Gewebe beinhaltet, in Reibungskontakt angewendet wird, das Gewebe mittels Anhaftung des Gewebes an dem Wundreinigungsmaterial von der Wundoberfläche zu entfernen, ohne feste Rückstände des Schaumstoffs an der Wundoberfläche zurückzulassen; **dadurch gekennzeichnet, dass**
der Schaumstoff eine Festigkeit von 30 Shore A Durometer bis 70 Shore A Durometer aufweist;
eine Kompressionsfestigkeit des Schaumstoffs 4,0 kPa bei 25% Kompression bis 9,0 kPa bei 50% Kompression ist; und
eine Dichte von 145 kg/m3 bis 270 kg/m3.

2. Wundreinigungsmaterial nach Anspruch 1, wobei der Schaumstoff ein Polymer ist, das aus der Gruppe ausgewählt ist, bestehend aus Polyvinylalkohol, Polyurethan, Polyolefin, Vinylacetat, Silikonelastomeren und deren Copolymeren.

3. Wundreinigungsmaterial nach Anspruch 1, wobei der Schaumstoff eine Vielzahl von Fasern aufweist, wobei jede einen Durchmesser von 5 Mikron bis 50 Mikron aufweist.

4. Wundreinigungsmaterial nach Anspruch 1, wobei die Flüssigkeit aus der Gruppe ausgewählt ist, bestehend aus Wasser, Kochsalzlösung und einem mehrwertigen Alkohol und Mischungen davon.

5. Wundreinigungsmaterial nach Anspruch 1, wobei die Flüssigkeit steril ist.

## Revendications

1. Matériau de débridement de plaie comprenant :
une mousse poreuse hydrophile solide à cellules ouvertes (100) imbibée d'un liquide de sorte que la mousse a une teneur en humidité de 20 % en poids à 60 % en poids ;
dans lequel la mousse poreuse solide (100) a une texture qui permet au matériau de débridement de plaie, lorsqu'il est appliqué en contact de friction en travers au moins d'une partie d'une surface de plaie d'un patient incluant le tissu à retirer, de retirer le tissu de la surface de plaie par adhérence du tissu au matériau de débridement de plaie sans laisser de débris solides de la mousse sur la surface de plaie ; **caractérisé en ce que**
la mousse a une rigidité de 30 duromètre Shore A à 70 duromètre Shore A ;
une rigidité en compression de la mousse est de 4,0 kPa à 25 % de compression à 0,0 kPa à 50 % de compression ; et
une densité de 145 kg/m³ à 270 kg/m³.

2. Matériau de débridement de plaie selon la revendication 1, dans lequel la mousse est un polymère sélectionné à partir du groupe constitué d'alcool polyvinylique, polyuréthane, polyoléfine, acétate de vinyle, élastomères de silicone et leurs copolymères.

3. Matériau de débridement de plaie selon la revendication 1, dans lequel la mousse comprend une pluralité de fibres ayant chacune un diamètre de 5 microns à 50 microns.

4. Matériau de débridement de plaie selon la revendication 1, dans lequel le liquide est sélectionné à partir du groupe constitué d'eau, de solution saline et d'alcool polyhydrique et des mélanges de ceux-ci.

5. Matériau de débridement de plaie selon la revendication 1, dans lequel le liquide est stérile.
